# EUROPEAN PATENT APPLICATION

(11) **EP 4 670 727 A1**
(43) Date of publication of application: **31.12.2025**
(21) Application number: 24760628.8
(22) Date of filing: 26.02.2024
(51) Int. Cl.: A61K 35/747, A61P 35/00, A61K 9/00

(54) **ANTI-CANCER ADJUVANT COMPRISING LACTOBACILLUS PLANTARUM STRAIN**

(30) Priority: 24.02.2023 KR 20230025173; 19.06.2023 KR 20230077911
(71) Applicant: GI Longevity Inc., Seongnam-si, Gyeonggi-do 13201 (KR)
(72) Inventor: JANG, Myung Ho, Seoul 05849 (KR); YANG, Bo Gie, Seoul 05849 (KR); KIM, A-Ram, Namyangju-si, Gyeonggi-do 12095 (KR)
(74) Representative: Vossius & Partner Patentanwälte Rechtsanwälte mbB
(86) International application number: PCT/KR2024/002433
(87) International publication number: WO 2024/177440

(57) **Abstract**

The present disclosure relates to an anticancer adjuvant including a *Lactobacillus plantarum* strain, and a bacterial cell of a *Lactobacillus plantarum* strain, or a culture broth of the strain, can overcome the resistance of cancer cells to an anticancer agent, increase the sensitivity of cancer cells to the anticancer agent, and ameliorate side effects caused by the anticancer agent when co-administered with a conventional anticancer agent, and thus can be effectively used as an anticancer adjuvant.

## Description

### Technical Field

The present disclosure relates to an anticancer adjuvant including a *Lactobacillus plantarum* strain.

### Background Art

Microbiome is a compound word of microbe and genome, and human microbiome refers to the genome of all microorganisms living in the human body. Several parts of the human body consist of various types of microorganisms. 70 % of the human microbiome is distributed in the digestive tract, with the largest number of microorganisms living in the large intestine. Since the microbiome is closely associated with the human immune system and physical development, a high correlation with various diseases, including metabolic diseases such as obesity and diabetes, inflammatory bowel disease, depression, and the like, has been consistently reported. After the Human genome project was initially completed in 2002, attempts were made to confirm the correlation between genes and incurable diseases, but it was confirmed that environmental factors, especially the microbiome environment, may have a greater impact on diseases. Microbiome therapeutic agents are living organisms that proliferate in the intestines and affect the human body through interactions with human cells. For this reason, the value thereof as innovative new drugs capable of improving the low efficacy and high recurrence rate of existing drugs for incurable diseases is attracting great attention. In addition, as the U.S. FDA approved in December 2022 REBYOTA^{®} from Ferring Pharmaceuticals, which is the first microbiome-based therapeutic agent for Clostridium difficile (C. difficile) infection (CDI), microbiome therapeutic agents started to be commercially available. Also, "SER-109" from Seres Therapeutics is under FDA review as the first oral microbiome therapeutic agent.

Gastrointestinal damage and dysfunction are well-known side effects of cancer chemotherapy treatment that can cause debilitation and are potentially life-threatening. In particular, chemotherapy administration is often associated with mucositis, diarrhea (chemotherapy-induced diarrhea (CID)), bacterial translocation, malabsorption, abdominal cramping, gastrointestinal bleeding, and vomiting. These side effects are clinical consequences of structural and functional damage to the intestinal epithelium, often requiring a reduction in chemotherapy dose and frequency, thus negatively affecting the patient's overall clinical outcomes. Intestinal mucositis and diarrhea may lead to severe dehydration, electrolyte imbalance, sepsis due to bacterial translocation, cardiovascular instability, and renal failure.

In particular, chemotherapy-induced damage to the small intestinal mucosa is referred to as gastrointestinal mucositis and is characterized by impaired absorption and barrier function of the small intestine. For example, widely used chemotherapeutic agents such as 5-fluorouracil (5-FU), irinotecan, and methotrexate are known to increase apoptosis in the small intestine of rodents, leading to villous atrophy and crypt hypoplasia. Furthermore, chemotherapeutic agents have been shown to increase apoptosis in intestinal crypts 24 hours after administration, followed by reductions in villus area, crypt length, number of mitoses per crypt, and enterocyte height 3 days after chemotherapy. Accordingly, structural changes within the small intestine may directly lead to intestinal dysfunction and, in some cases, diarrhea. These are inherently untreatable, progressive problems once established, although these are gradually relieved after cancer chemotherapy.

Recently, combination therapies that enhance the effectiveness of treatment for diseases have been emerging. Combination therapy is the use of two or more drugs or methods simultaneously or in relatively rapid succession. In combination therapy, the side effects of two or more treatment methods used may be additive or less than additive, while the therapeutic effects may be additive or greater than additive. The effect that occurs when each component is administered in combination can be expected to be a synergistic effect, exceeding the sum of the effects when administered alone as a single component. Therefore, there is a need to develop combination therapy that can synergistically increase the effects of treatments each using a single ingredient.

### Technical Problem

An aspect is to provide a composition for use as an adjuvant to anticancer treatment, including a *Lactobacillus plantarum* strain, a culture of the strain, a lysate of the strain, or a mixture thereof.

Another aspect is to provide a composition for enhancing sensitivity to an anticancer agent, the composition including a *Lactobacillus plantarum* strain, a culture of the strain, a lysate of the strain, or a mixture thereof.

Another aspect is to provide an anticancer adjuvant including a *Lactobacillus plantarum* strain, a culture of the strain, a lysate of the strain, or a mixture thereof.

Another aspect is to provide a method of preventing or reducing a side effect in a subject administered with an anticancer agent, the method including administering, to the subject administered with an anticancer agent, a *Lactobacillus plantarum* strain, a culture of the strain, a lysate of the strain, or a mixture thereof.

Another aspect is to provide use of a *Lactobacillus plantarum* strain, a culture of the strain, a lysate of the strain, or a mixture thereof for preventing or reducing a side effect in a subject administered with an anticancer agent.

Another aspect is to provide use of a composition including a *Lactobacillus plantarum* strain, a culture of the strain, a lysate of the strain, or a mixture thereof for enhancing sensitivity to an anticancer agent.

Another aspect is to provide a method of enhancing sensitivity to an anticancer agent, including administering a composition including a *Lactobacillus plantarum* strain, a culture of the strain, a lysate of the strain, or a mixture thereof to a subject in need thereof.

Another aspect is to provide use of a composition including a *Lactobacillus plantarum* strain, a culture of the strain, a lysate of the strain, or a mixture thereof for the preparation of a pharmaceutical formulation for enhancing sensitivity to an anticancer agent.

Another aspect is to provide use of a composition including a *Lactobacillus plantarum* strain, a culture of the strain, a lysate of the strain, or a mixture thereof as an adjuvant to anticancer treatment.

Another aspect is to provide a method for use as an adjuvant to anticancer treatment, including administering an effective amount of a composition including a *Lactobacillus plantarum* strain, a culture of the strain, a lysate of the strain, or a mixture thereof to a subject in need thereof.

Another aspect is to provide use of a composition including a *Lactobacillus plantarum* strain, a culture of the strain, a lysate of the strain, or a mixture thereof for the preparation of a pharmaceutical formulation as an adjuvant to anticancer treatment.

### Solution to Problem

An aspect provides a composition for use as an adjuvant to anticancer treatment or a composition for enhancing sensitivity to an anticancer agent, each composition including a strain of the genus *Lactobacillus,* particularly a *Lactobacillus plantarum* GB104 strain, as an active ingredient.

*Lactobacillus* is an aerobic or facultative anaerobic, gram-positive *bacillus* widely distributed in nature. Microorganisms belonging to the genus *Lactobacillus* include *Lactobacillus plantarum, Lactobacillus sakei,* and the like. As a result of having researched to develop a novel strain with an excellent anticancer effect, the inventors of the present disclosure selected *Lactobacillus plantarum* GB104 as an anticancer candidate strain. The strain was deposited in the Korea Collection for Type Cultures of Korea Research Institute of Bioscience and Biotechnology under accession number KCTC14107BP on January 14, 2020. The strain belongs to a probiotic strain, is harmless to the human body, and may be used without side effects.

The name of *Lactobacillus* has been changed to *Limosilactobacillus* or *Lactiplantibacillus,* and the changed strain names in this specification may be used interchangeably. For example, the strain name *Lactobacillus plantarum* was changed to *Lactiplantibacillus plantarum.*

The term *"Lactobacillus plantarum* GB104" as used herein may be interchangeably described as a *L. plantarum* GB104 strain or a *Lactobacillus plantarum* GB104 strain (Accession No.: KCTC14107BP).

In an embodiment, the strain may be a strain deposited under accession number KCTC 14107BP.

In an embodiment, the strain may be a strain including a 16S rRNA gene consisting of the nucleotide sequence of SEQ ID NO: 1.

In an embodiment, the strain may be a strain having 16S rRNA consisting of the nucleotide sequence of SEQ ID NO: 1 or 16s rRNA including a nucleotide sequence with 97 % or more nucleotide sequence identity therewith. Specifically, the strain may have at least 93 %, 95 %, 96 %, 97 %, 98 %, 99 %, 99.5 %, 99.8 %, 99.9 % or 100 % homology to the nucleotide sequence consisting of SEQ ID NO: 1 of this specification.

In an embodiment, the strain may be a mutant of a naturally occurring *Lactobacillus plantarum* strain.

In an embodiment, the strain may be a live bacterium, a dead bacterium, or a cytoplasmic fraction obtained by lysing the strain, and may preferably be a live bacterium.

The term "culture" as used herein may be used interchangeably with "culture supernatant," "conditioned culture broth," or "conditioned medium," and may refer to the entire medium including: a strain obtained by culturing a strain of the genus *Lactobacillus* in a medium capable of supplying nutrients to the strain to grow and survive *in vitro* for a certain period of time; metabolites thereof; extra nutrients; and the like. The culture refers to a product obtained by culturing a probiotic strain in a known medium, and the product may or may not include the strain itself. The medium may be selected from known liquid media or solid media, and for example, may be, but is not limited to, an MRS liquid medium, a GAM liquid medium, an MRS agar medium, a GAM agar medium, and a BL agar medium.

The term "lysate" as used herein may be used interchangeably with "lysate solution," and may refer to a solution or suspension in an aqueous medium of crushed cells of a microorganism such as *Lactobacillus plantarum.* A cell lysate includes, for example, macromolecules such as DNA, RNA, proteins, peptides, carbohydrates, or lipids and/or micromolecules such as amino acids, sugars, or fatty acids, or fractions thereof. The lysate also includes cell debris, which may be smooth or have a granular structure.

The culture broth may include a culture broth itself obtained by culturing a strain, or a concentrate or freeze-dried product thereof, or a culture supernatant obtained by removing the strain from the culture broth, or a concentrate or freeze-dried product thereof.

The culture broth may be obtained by culturing *Lactobacillus plantarum* in an appropriate medium (e.g., MRS plate medium) at any temperature exceeding 10 °C or less than 40 °C for a certain period of time, for example, 4 to 50 hours.

In an embodiment, the strain may upregulate the expression of a tight junction in intestinal tissue. Specifically, the strain may upregulate the expression of any one or more selected from the group consisting of tight junction proteins claudin-1, claudin-2, claudin-4, claudin-5, zonula occludens (ZO)-1, and occludin in intestinal tissue.

In an embodiment, the strain may be for the reduction, amelioration, prevention or treatment of a side effect caused by an anticancer agent.

The strain or a mixture including the same, of the present disclosure, may be used to reduce the toxicity of a wide variety of anticancer agents (e.g., chemotherapeutic agents) or accompanying chemotherapy treatments. Although some anticancer agents or chemotherapies are known to cause chemotherapy-induced diarrhea and damage gastrointestinal mucosa, the present disclosure may also be used to reduce the asymptomatic occurrence of CID or gastrointestinal mucosal damage from substantially all anticancer agents or chemotherapies.

The term "sensitivity to an anticancer agent" as used herein refers to the degree to which cancer cells respond to an anticancer agent. This is because the therapeutic effect of an anticancer agent on cancer cells becoming resistant to the anticancer agent, which exhibits reduced efficacy due to repeated dosing or administration, is reduced. When the anticancer agent is administered in combination with the *Lactobacillus plantarum* strain, the activity of the anticancer agent against anticancer agent-resistant cancer may further be enhanced.

The term "reduction" as used herein refers to any action that reduces side effects of the anticancer agent via administration of the anticancer adjuvant, and the term "amelioration" as used herein refers to any action that reduces side effects of the anticancer agent via administration of the anticancer adjuvant or any action that improves or beneficially changes the symptoms of cancer due to the reduction in the side effects of the anticancer agent.

The term "treating" as used herein refers to any form of treatment or prevention that provides a subject suffering from a disease or at risk of developing a disease with effects including improving condition (e.g., one or more symptoms) of the subject, delaying disease progression, delaying the onset of symptoms, slowing symptom progression, and the like. Therefore, the term "treatment" also includes prophylactic treatment of a subject to prevent the occurrence of symptoms.

The terms "treatment" and "prevention" as used herein are not intended to mean cure or complete elimination of symptoms. These terms refer to any form of treatment that provides an effect to a patient suffering from a disease, including improvement of condition of a patient (e.g., one or more symptoms), delay in disease progression, and the like.

The term "therapeutically effective amount" as used herein refers to an amount sufficient to produce a desired effect in a patient with cancer, including reduction of side effects of anticancer treatment, improvement of condition (e.g., one or more symptoms), delay in disease progression, or the like.

The term "cancer" as used herein typically refers to a physiological condition in an animal, characterized by abnormal or uncontrolled cell growth. For example, cancer and cancer pathology may be related to metastasis, interference with normally functioning surrounding cells, release of cytokines or other secreted products at abnormal levels, inhibition or enhancement of inflammatory or immunological responses, neoplasia, premalignancy, malignancy, invasion into surrounding or distant tissues or organs, such as lymph node, and the like.

The cancer may be gastrointestinal cancer or non-gastrointestinal cancer.

The gastrointestinal cancer is a malignant tumor occurring in the gastrointestinal tract such as esophagus, stomach, small intestine, or large intestine. For example, the gastrointestinal cancer may be, but is not limited to, one or more cancers selected from the group consisting of esophageal cancer, gallbladder cancer, liver cancer, biliary tract cancer, pancreatic cancer, gastric cancer, small intestine cancer, colorectal cancer, colon cancer, anal cancer, and rectal cancer. In an embodiment, the gastrointestinal cancer may be colorectal cancer.

The non-gastrointestinal cancer includes malignant tumors occurring in organs other than the gastrointestinal tract or digestive system without limitation, and for example, may be, but is not limited to, leukemia, acute myeloid leukemia, neuroblastoma, retinoblastoma, lung cancer, head and neck cancer, salivary gland cancer, melanoma, laryngeal cancer, prostate cancer, breast cancer, bladder cancer, kidney cancer, multiple myeloma, cervical cancer, thyroid cancer, ovarian cancer, urethral cancer, skin cancer, osteosarcoma, glioblastoma, brain tumor, or lymphoma.

In an embodiment of the present disclosure, the cancer may be colorectal cancer, and the colorectal cancer includes malignant tumors occurring in one or more sites selected from the group consisting of ascending colon, transverse colon, descending colon, sigmoid colon, and rectal mucosa. The colorectal cancer may be, but is not limited to, one or more types selected from the group consisting of adenocarcinoma, lymphoma, malignant carcinoid, leiomyosarcoma, Kaposi's sarcoma, and squamous cell carcinoma.

The anticancer agent may be selected from the group consisting of a chemotherapeutic agent for chemotherapy, which is conventional therapy that can be used in combination, a targeted anticancer agent, a cancer immunotherapeutic agent, and a combination thereof.

The term "chemotherapeutic agent" as used herein is also referred to as an antineoplastic agent or a cytotoxic agent. The chemotherapeutic agent is a generic term for drugs that exhibit anticancer activity mainly by acting directly on DNA to block DNA replication, transcription, and translation processes, or by interfering with the synthesis of nucleic acid precursors in the metabolic pathway and by inhibiting cell division. The antineoplastic agent exhibits cytotoxicity by acting not only on tumor cells but also on normal cells. The chemotherapeutic agent may be used in maintenance therapy. In addition, the term "maintenance therapy" as used herein refers to treatment of cancer with drugs after initial anticancer treatment, and refers to a treatment method performed to prevent or delay cancer recurrence.

Specifically, the chemotherapeutic agent may be any one selected from the group consisting of an alkylating agent, a microtubule inhibitor, an antimetabolite, and a topoisomerase inhibitor. The alkylating agent may be any one selected from the group consisting of mechlorethamine, cyclophosphamide, ifosfamide, melphalan, chlorambucil, thiotepa, altretamine, procarbazine, busulfan, streptozotocin, carmustine, lomustine, dacarbazine, cisplatin, carboplatin, and oxaliplatin. The microtubule inhibitor may be any one selected from the group consisting of docetaxel, velban, oncovin, and navelbine. The antimetabolite may be any one selected from the group consisting of fluorouracil, capecitabine, cytarabine, gemcitabine, fludarabine, methotrexate, pemetrexed, and mercaptopurine. The topoisomerase inhibitor may be any one selected from the group consisting of hycamtin, camptosar, vepesid, paclitaxel, blenoxane, adriamycin, and cerubidine.

The term "targeted anticancer agent" as used herein refers to a therapeutic agent that specifically kills cancer cells by targeting specific proteins or specific genetic changes that are abundantly present or frequently occur only in cancer cells and blocking signals involved in the growth and development of cancer. The targeted anticancer agent is classified into monoclonal antibodies that act extracellularly, and small molecule substances that act intracellularly. Monoclonal antibodies are anticancer agents that block cancer cell-inducing signals transmitted to the outside of cells, and act on initiation signals related to proliferation, death, and the like, and small molecule substances act on complex signal transduction occurring inside cells.

Specifically, proteins to be targeted may be EGFR, VEGFR, CD20, CD38, RNAK-L, BTK, Bcr-abl, PDGFR/FGFR family, MEK/RAF, HER2/Neu, ubiquitin, JAK, MAP2K, ALK, PARP, TGFβRI, proteasome, Bcl-2, C-Met, VR1, VR2, VR3, c-kit, AXL, RET, Braf, DNMT, CDK4/6, STING, and the like.

The term "cancer immunotherapeutic agent" as used herein is a substance that inhibits the activity of an immune checkpoint protein that inhibits the differentiation, proliferation, and activity of immune cells, and is known to eradicate cancer cells by preventing the cancer cells from exerting the function of evading the immune system. The cancer immunotherapeutic agent may be any one selected from the group consisting of an anti-CTLA-4 antibody, an anti-PD-1 antibody, an anti-PD-L1 antibody, an anti-PD-L2 antibody, an anti-B7-H4 antibody, an anti-HVEM antibody, an anti-TIM3 antibody, an anti-GAL9 antibody, an anti-LAG3 antibody, an anti-VISTA antibody, an anti-KIR antibody, an anti-BTLA antibody, and an anti-TIGIT antibody. In an embodiment, the cancer immunotherapeutic agent may be, but is not limited to, ipilimumab, pembrolizumab, nivolumab, cemiplimab, atezolizumab, avelumab, and durvalumab.

In an embodiment, the chemotherapeutic agent may be, but is not limited to, any one selected from the group consisting of mechlorethamine, ifosfamide, melphalan, chlorambucil, thiotepa, altretamine, procarbazine, busulfan, streptozocin, carmustine, lomustine, dacarbazine, cisplatin, carboplatin, oxaliplatin, docetaxel, Velban, Oncovin, Navelbine, 5-fluorouracil, capecitabine, cytarabine, gemcitabine, fludarabine, methotrexate, pemetrexed, mercaptopurine, Hycamtin, irinotecan, camptosar, folinic acid, leucovorin, Vepesid, paclitaxel, Blenoxane, Adriamycin, Cerubidine, trifluridine/tipiracil, and regorafenib, or a combination thereof. Specifically, the anticancer agent may be irinotecan.

In an embodiment, the chemotherapeutic agent may be, but is not limited to, any one selected from the group consisting of mechlorethamine, cyclophosphamide, chlorambucil, ifosfamide, Melphalan, thiotepa, altretamine, procarbazine, busulfan, streptozocin, carmustine, lomustine, dacarbazine, cisplatin, carboplatin, oxaliplatin, vinblastine, vincristine, vinorelbine, fluorouracil (5-FU), capecitabine, cytarabine, gemcitabine, fludarabine, methotrexate, pemetrexed, mercaptopurine (6-MP), topotecan, irinotecan, etoposide, paclitaxel, docetaxel, etoposide, dactinomycin, doxorubicin, daunorubicin, mitomycin, bleomycin, bleosein, bevacizumab, cetuximab, rituximab, trastuzumab, panitumumab, aflibercept, rituximab, pembrolizumab, nivolumab, atezolizumab, duvalumab, avelumab, ipilimumab, gefitinib, erlotinib, osimertinib, lazertinib, afatinib, crizotinib, alectinib, brigatinib, sorafenib, sunitinib, pazopanib, lenvatinib, axitinib, cabozantinib, imatinib, dasatinib, nilotinib, midostaurin, palbociclib, ribociclib, abemaciclib, olaparib, trifluridine/tipiracil, and regorafenib, or a combination thereof.

In an embodiment, the targeted anticancer agent may be, but is not limited to, any one selected from the group consisting of abciximab, adalimumab, basiliximab, bezlotoxumab, canakinumab, daclizumab, denosumab, efalizumab, golimumab, inflectra, natalizumab, olaratumab, omalizumab, palivizumab, panitumumab, trastuzumab, pertuzumab, aflibercept, ramucirumab, cetuximab, rituximab, tocilizumab, secukinumab, ustekinumab, bevacizumab, avelumab, CDNs, SB11285, and DMXAA, or a combination thereof.

In an embodiment, the cancer immunotherapeutic agent may be, but is not limited to, any one selected from the group consisting of avelumab, durvalumab, nivolumab, abagovomab, adecatumumab, atezolizumab, afutuzumab, alemtuzumab, anatumomab mafenatox, apolizumab, blinatumomab, BMS-936559, catumaxomab, cemiplimab, epacadostat, epratuzumab, indoximod, inotuzumab, ozogamicin, intelumumab, pembrolizumab, ipilimumab, isatuximab, lambrolizumab, MED 14736, MPDL3280A, obinutuzumab, ocaratuzumab, ofatumumab, olaratumab, pidilizumab, rituximab, ticilimumab, samalizumab, and tremelimumab, or a combination thereof.

In an embodiment, the side effects caused by the anticancer agent may be selected from the group consisting of vomiting, oral mucositis, colitis, ulcerative colitis, diarrhea, enteritis-induced diarrhea, constipation, esophagitis, bleeding, alopecia, infection, fever, thrombocytopenia, anemia, abdominal pain, peripheral neurotoxicity, central neurotoxicity, muscle pain, bone pain, decreased activity, lethargy, decreased appetite, weight loss, fatigue, decreased food intake, nephrotoxicity, splenic toxicity, thymic toxicity, hepatotoxicity, cardiotoxicity, pulmonary toxicity, decreased motor function, immunotoxicity, and inflammation, and specifically, may be, but are not limited to, vomiting, oral mucositis, colitis, ulcerative colitis, diarrhea, enteritis-induced diarrhea, constipation, esophagitis, decreased activity, lethargy, decreased appetite, weight loss, fatigue, decreased food intake, and inflammation. Specifically, the chemotherapeutic agents have a mechanism that inhibits DNA division in rapidly proliferating cells, and thus affect not only cancer cells but also normal cells, resulting in the occurrence of various side effects.

In an embodiment, the strain may inhibit or ameliorate side effects caused by anticancer agents (e.g., chemotherapeutic agents), specifically, intestinal inflammation responses or intestinal tissue damage induced by anticancer agents, or weakening or destruction of a tight junction in intestinal tissue induced by anticancer agents. In addition, the strain may inhibit or ameliorate functional disorders such as decreased activity (asthenia), vomiting, decreased appetite, weight loss, constipation, stomatitis, and esophagitis induced by anticancer drugs.

In an embodiment of the present disclosure, when the *Lactobacillus plantarum* GB104 strain and irinotecan, which is a chemotherapeutic agent, were co-administered to a tumor animal model transplanted with mouse colorectal cancer cell line CT26 or MC-38, the effect of alleviating or ameliorating side effects caused by the chemotherapeutic agent irinotecan was exhibited. Specifically, compared to a mouse colorectal cancer model administered with irinotecan as a control, a group co-administered with the *Lactobacillus plantarum* GB104 strain and irinotecan showed improvements of 50 % or greater in all indicators of the survival rate, diarrhea, activity level, coat appearance, and posture of mice.

In an embodiment, in a tumor animal model transplanted with mouse colorectal cancer cell line CT26 or MC-38, decreased activity, induction of inflammatory responses in intestinal tissue, damage to intestinal tissue (e.g., intestinal crypt), and weakening or destruction of a tight junction in intestinal tissue were observed as side effects caused by irinotecan, which is a chemotherapeutic agent. Thus, before, during, and after administration (e.g., intraperitoneal administration) of irinotecan, the *Lactobacillus plantarum* GB104 strain was intraperitoneally administered, through which the effect of inhibiting, ameliorating, or alleviating the side effects was confirmed. In particular, the degree of inflammation and tissue damage in intestinal tissue and the expression level of a tight junction in intestinal tissue showed improvement effects that were equivalent to or better than those of a mouse group without induced colorectal cancer.

In an embodiment, assuming that symptoms appearing as side effects in a group administered with the anticancer agent alone as compared to a control not administered with the anticancer agent are set to 100 %, the *Lactobacillus plantarum* GB104 strain, when administered before, simultaneously with, or after the anticancer agent, may reduce the side effects caused by the anticancer agent to 90 % or less, 80 % or less, 70 % or less, 67 % or less, 10 to 90 %, 10 to 80 %, 10 to 70 %, 10 to 67 %, 20 to 90 %, 20 to 80 %, 20 to 70 %, 20 to 67 %, 30 to 90 %, 30 to 80 %, 30 to 70 %, 30 to 67 %, 40 to 90 %, 40 to 80 %, 40 to 70 %, 40 to 67 %, 50 to 90 %, 50 to 80 %, 50 to 70 %, 50 to 67 %, or 60 to 67 %.

The composition of the present disclosure may be prepared by including one or more pharmaceutically acceptable carriers in addition to the above-described active ingredients for administration. As the pharmaceutically acceptable carrier, saline, sterile water, Ringer's solution, buffered saline, dextrose solution, maltodextrin solution, glycerol, ethanol, liposomes, and a mixture of one or more of these components may be used. If needed, other conventional additives such as antioxidants, buffers, and bacteriostats may be added. In addition, the pharmaceutical composition may be formulated as injectable formulations such as an aqueous solution, a suspension, and an emulsion, pills, capsules, granules, or tablets by additionally adding a diluent, a dispersant, a surfactant, a binder, and a lubricant. In addition, target organ-specific antibodies or other ligands may be used in combination with the carrier to act specifically on a target organ. Furthermore, the pharmaceutical composition may be preferably formulated according to each disease or ingredient by using an appropriate method in the art or a method disclosed in the Remington's document (Remington's Pharmaceutical Science (latest edition), Mack Publishing Company, Easton PA).

In an embodiment, the strain and the anticancer agent may be co-administered simultaneously, sequentially, or in reverse order.

The term "combined therapy," "co-administration," or "in combination," as used herein, refers to any form of simultaneous or concurrent treatment using at least two separate anticancer agents and anticancer adjuvants. Ingredients of the combined therapy may be administered simultaneously, sequentially, or in any order. The ingredients may be appropriately administered at different dosages, different frequencies of administration, or via different routes.

Specifically, for the co-administration, the *Lactobacillus plantarum* strain and the anticancer agent may be administered simultaneously, or the *Lactobacillus plantarum* strain may be administered, followed by administration of the anticancer agent. The combined therapy according to the present disclosure may be defined as being capable of providing a synergistic effect if the efficacy measured through, for example, the degree of response, the rate of response, time to disease progression, or the survival period is therapeutically superior to the efficacy obtained by administering one or the other of the ingredients of the combined therapy at conventional doses. For example, if the efficacy thereof is therapeutically superior to the efficacy obtained using each of the ingredients alone, the efficacy of the combined therapy is synergistic. In particular, it is considered that there is a synergistic effect if conventional doses of the *Lactobacillus plantarum* strain and the anticancer agent can be reduced while the side effects are reduced/reduced or small, compared with those occurring when each ingredient is used at a conventional dose, without adversely affecting one or more of the degree of response, the rate of response, time to disease progression, and survival data, particularly the response duration.

The term "administered simultaneously" as used herein is not particularly limited and means that the ingredients of the combined therapy are substantially administered at the same time, e.g., as a mixture or in immediate subsequent order.

The term "administered sequentially" as used herein is not particularly limited and means that the ingredients of the combined therapy are not administered simultaneously, but are administered one by one or in clusters with a specific time interval between administrations. The time interval may be the same or different between the respective administrations of the ingredients of the combined therapy and may be selected, for example, from the range of 2 minutes to 96 hours, 1 day to 7 days, or one, two or three weeks. Generally, the time interval between administrations may be in the range of several minutes to several hours, such as in the range of 2 minutes to 72 hours, 30 minutes to 24 hours, or 1 hour to 12 hours. Further examples thereof include time intervals in the range of 24 to 96 hours, 12 to 36 hours, 8 to 24 hours, and 6 to 12 hours.

In an embodiment, the strain and/or the anticancer agent may be administered to a mammal, including a human, via various routes. The strain and/or the anticancer agent may be administered using any commonly used method, and for example, may be administered intratumorally, intraarterially, intravenously, intravascularly, intrapleurally, intraperitoneally, intratracheally, intrathecally, intramuscularly, endoscopically, intralesionally, transdermally, subcutaneously, regionally, stereotactically, orally, or by direct injection or perfusion, and specifically, may be administered orally, intravenously, or subcutaneously. The administration methods of the strain and/or the anticancer agent may differ from each other. Specifically, the strain may be administered orally, and the anticancer agent may be administered by intraperitoneal injection.

The composition according to an embodiment may include 0.001 wt% to 80 wt% of the *Lactobacillus plantarum* strain with respect to a total weight of the composition. In other embodiments, the dose of the *Lactobacillus plantarum* strain may range from 0.01 mg to 10,000 mg, 0.1 mg to 1000 mg, 1 mg to 100 mg, 0.01 mg to 1000 mg, 0.01 mg to 100 mg, 0.01 mg to 10 mg, or 0.01 mg to 1 mg. The strain may be included in the composition in a therapeutically effective amount or at a nutritionally effective concentration. For example, the strain may be included in an amount of 10³ to 10¹⁶ CFU/g, 10³ to 10¹⁵ CFU/g, 10³ to 10¹⁴ CFU/g, 10³ to 10¹³ CFU/g, 10³ to 10¹² CFU/g, 10⁴ to 10¹⁶ CFU/g, 10⁴ to 10¹⁵ CFU/g, 10⁴ to 10¹⁴ CFU/g, 10⁴ to 10¹³ CFU/g, 10⁴ to 10¹² CFU/g, 10⁵ to 10¹⁶ CFU/g, 10⁵ to 10¹⁵ CFU/g, 10⁵ to 10¹⁴ CFU/g, 10⁵ to 10¹³ CFU/g, 10⁵ to 10¹² CFU/g, 10⁶ to 10¹³ CFU/g, 10⁶ to 10¹² CFU/g, 10⁷ to 10¹³ CFU/g, 10⁷ to 10¹² CFU/g, 10⁸ to 10¹³ CFU/g, or 10⁸ to 10¹² CFU/g, or may be included in the composition as a culture of an equal number of viable or non-viable bacteria. Specifically, for adult patients, 1 x 10³ to 1 x 10¹⁶ CFU/g of live or dead bacteria may be administered in single or multiple doses. A dose may vary depending on various factors such as formulation method, administration method, age, body weight, and gender of a patient, pathological conditions, diet, administration time, administration route, excretion rate, response sensitivity, and the dose may be appropriately adjusted by those of ordinary skill in the art in consideration of these factors. The pharmaceutical composition may be administered once or administered two or more times to the extent that adverse effects are clinically acceptable, and may be administered to one site or two or more sites. For non-human animals, the pharmaceutical composition may be administered at the same dose as that for humans per kg (body weight), or may be administered, for example, in an amount obtained by converting the dose on the basis of a volume ratio (e.g., average value) of organs (heart or the like) between a target animal and humans. Possible routes of administration may include oral, sublingual, parenteral (e.g., subcutaneous, intramuscular, intraarterial, intraperitoneal, intrathecal, or intravenous), rectal, topical (including transdermal), inhalation, and injection, or insertion of an implantable device or substance. Examples of animals to be treated according to an embodiment include humans and other target mammals, and specific examples thereof include humans, monkeys, mice, rats, rabbits, sheep, cows, dogs, horses, pigs, and the like. **In an** embodiment, the composition includes a killed dry strain, and may be administered at a dose of 1 g to 10 g, 0.5 g to 1.5 g, 2.5 g to 3.5 g, or 4.5 g to 5.5 g once to 3 times a day.

Another aspect provides an anticancer adjuvant including a *Lactobacillus plantarum* strain, a culture of the strain, a lysate of the strain, or an extract or mixture of the strain, the culture, and the lysate.

The "strain," "anticancer agent," "administration," and "co-administration" are as described above.

The subject may be a subject suffering from cancer. **In** other embodiments, the subject may be a mammal, and preferably, may be a human.

The term "anticancer therapy adjuvant" or "anticancer adjuvant" as used herein refers to an agent capable of improving, enhancing or increasing the anticancer effect of an anticancer agent. Generally, an anticancer adjuvant refers to an agent that does not exhibit anticancer activity by itself, but is capable of improving, enhancing, or increasing the anticancer effect of an anticancer agent when used in combination with the anticancer agent. In contrast, the anticancer adjuvant according to an aspect has the effect of enhancing anticancer activity via co-administration with an anticancer agent.

The administration route, dosage, and number of administrations of the *Lactobacillus plantarum* GB104 strain and the anticancer agent may be administered to a subject in various ways and amounts depending on the condition of a patient, and the presence or absence of side effects, and the optimal administration method, dosage and number of administrations may be appropriately selected within appropriate ranges by one of ordinary skill in the art. In other embodiments, the composition may be administered in combination with other drugs known to have therapeutic effects on cancer (e.g., anticancer agents described above) or physiologically active substances, in addition to the active ingredients described above, or may be formulated in the form of a combination formulation with other drugs.

In an embodiment, the *Lactobacillus plantarum* GB104 strain may be formulated as an anticancer adjuvant together with a pharmaceutically acceptable carrier or an excipient. Specifically, the strain may be combined as a pharmaceutical composition together with another compound effective in ameliorating or preventing side effects of cancer chemotherapy. The formulation used in the method of the present disclosure may be conveniently provided in unit dosage form and may be prepared by methods known in the art. The amount of the active ingredient that may be combined with a carrier material to produce a single dosage form will vary depending on a host being treated and the particular mode of administration. The amount of the active ingredient that may be combined with a carrier material to produce a single dosage form will generally be the amount of a compound that produces a therapeutic effect.

Generally, formulations are prepared using liquid carriers, finely divided solid carriers, or both, and then a product may be molded as needed. Pharmaceutical compositions suitable for parenteral administration may include the *Lactobacillus plantarum* GB104 strain in combination with one or more pharmaceutically acceptable sterile isotonic aqueous or non-aqueous solutions, dispersions, suspensions or emulsions, or sterile powders which may be reconstituted into sterile injectable solutions or dispersions immediately before use.

Another aspect provides a method of preventing or reducing colon damage in a subject administered with an anticancer agent, including administering, to the subject administered with an anticancer agent, a *Lactobacillus plantarum* strain, a culture of the strain, a lysate of the strain, or an extract or mixture of the strain, the culture, or the lysate.

In an embodiment, the *Lactobacillus plantarum* GB104 strain and the anticancer agent (e.g., a chemotherapeutic agent) may preferably be co-administered for 2 days or longer, and specifically, administration of the strain may start before the anticancer agent starts to be administered, may start simultaneously with the anticancer agent, or may start after administration of the anticancer agent is completed.

The terms, methods, and the like described for the disclosure apply equally to pharmaceutical compositions, methods, uses, and the like as long as these do not contradict each other.

### Advantageous Effects of Invention

A bacterial cell of a *Lactobacillus plantarum* strain, or a culture broth of the strain, can overcome the resistance of cancer cells to a targeted anticancer agent, increase the sensitivity of cancer cells to the anticancer agent, and ameliorate side effects caused by the anticancer agent when co-administered with a conventional targeted anticancer agent, and thus can be effectively used as an anticancer adjuvant.

### Brief Description of Drawings

FIG. 1 is a graph showing the activity levels of mice upon administration of a GB104 strain in a mouse colorectal cancer (CT26) model.
FIG. 2 is a graph showing the coat appearance of mice upon administration of a GB104 strain in a mouse colorectal cancer (CT26) model.
FIG. 3 is a graph showing the severity of diarrhea in mice upon administration of a GB104 strain in a mouse colorectal cancer (CT26) model.
FIG. 4 is a graph showing the posture of mice upon administration of a GB104 strain in a mouse colorectal cancer (CT26) model.
FIG. 5 is a graph showing the survival rate of mice upon administration of a GB104 strain in a mouse colorectal cancer (CT26) model.
FIG. 6 is a graph showing the effect of alleviating decreased activity (lethargy) in mice upon administration of a GB104 strain in a mouse colorectal cancer (MC-38) model.
FIG. 7 is a graph showing the effect of alleviating inflammation and damage in the intestinal tissues of mice upon administration of a GB104 strain in a mouse colorectal cancer (MC-38) model.
FIG. 8 is a graph showing the upregulation of tight junction expression in the intestinal tissues of mice upon administration of a GB104 strain in a mouse colorectal cancer (MC-38) model.

### Mode for the Invention

Hereinafter, preferred examples will be presented to aid in understanding of the present disclosure. However, the following examples are provided only to more easily understand the present disclosure, and the content of the present disclosure is not limited by the following examples. Various modification can be made in examples, and thus examples are not limited to the examples disclosed below and can be implemented in various other forms.

### Example 1. Isolation and identification of Lactobacillus plantarum GB104 strain

The isolation and identification of a *Lactobacillus plantarum* GB104 strain were performed by methods described in Korean Patent Application No. 10-2020-0186738 and Korean Patent Application No. 10-2022-0080567. The above-described documents are incorporated herein by reference in their entirety.

Briefly, *Lactobacillus plantarum* GB104 was isolated from vaginal samples of healthy women who visited a hospital for the purpose of health examination. First, vaginal internal samples were taken with a cotton swab, pre-inoculated on a Rogosa SL (MRS) plate medium, and incubated in an anaerobic chamber at 37 °C for 48 hours. When bacterial colonies grew, single colonies were subcultured onto fresh MRS plate media for pure isolation. After pure isolation, strains were cultured using MRS medium. Next, among the cultured strains, a *Lactobacillus plantarum* GB104 strain, which showed low cytotoxicity and inhibitory effect on fat cell accumulation, was finally selected. To identify the finally selected *Lactobacillus plantarum* GB104 strain, the 16S rRNA gene sequences obtained through PCR using primers targeting the 16S rRNA gene were analyzed by the Sanger sequencing method, and the 16s rRNA sequences of *Lactobacillus plantarum* GB104 were represented as SEQ ID NO: 1. The inventors of the present disclosure named the GB104 strain as *"Lactobacillus plantarum* GB104" (accession number: KCTC14107BP) and deposited the strain in the Korean Collection for Type Cultures (KCTC) located in the Korea Research Institute of Bioscience and Biotechnology on January 14, 2020. In addition, the strain name *Lactobacillus plantarum* was changed to *Lactiplantibacillus plantarum.* In the following examples, the changed strain names of existing strains are described interchangeably.

### Experimental Example 1. Effect of L. plantarum GB104 strain on alleviating side effects caused by anticancer agent in mouse colorectal cancer model

The effect of an *L. plantarum* GB104 strain on alleviating various side effects upon administration of a high dose of irinotecan, which is used as a primary anticancer therapeutic agent for patients with metastatic colorectal cancer, was examined in a syngeneic CT26 colorectal carcinoma model.
Group 1 (G1): PBS-administered mice in a mouse colorectal cancer model
Group 2 (G2): Mice administered with GB104 alone in a mouse colorectal cancer model
Group 3 (G3): Mice administered with irinotecan and PBS in a mouse colorectal cancer model
Group 4 (G4): Mice administered with irinotecan and GB104 in a mouse colorectal cancer model

Five-week-old BALB/c mice were purchased and acclimatized for one week, and experiments were conducted at 6 weeks of age after hair removal from the right flank. A tumor model was established by subcutaneously injecting the BALB/c-derived colorectal cancer cell line CT26 into the right flank of each mouse at a concentration of 5 x 10⁵ cells/100 µL per mouse. Tumor volume was measured using a digital caliper and calculated by using tumor volume (mm³) (width² x length)/2. From the time of tumor cell injection, the *L. plantarum GB104* strain was orally administered daily to the animal model at a concentration of 1 x 10⁹ CFU per mouse until immediately before the experiments were completed. The chemotherapeutic agent irinotecan (400 mg/kg) was administered intraperitoneally every other day on days 7, 9, and 11 after tumor cell injection.

Side effects caused by irinotecan administration were evaluated using a mouse wellbeing score protocol adapted from UK Co.-ordinating Committee on Cancer Research, 1988. The specific mouse wellbeing score criteria are shown in Table 1 below.

**Table 1**

| Mouse wellbeing score | | |
|---|---|---|
| Mouse activity level (movement) | 2 | Move around normally |
| | 1 | Move slowly or less frequently and with an altered gait |
| | 0 | not moving and taking no more than 5 steps |
| Coat (smoothness) | 2 | Healthy, smooth uninterrupted coat |
| | 1 | Slightly fluffy coat |
| | 0 | Severe fluffy coat with evident gaps of visible skin |
| Diarrhea (stool) | 0 | Normal |
| | 1 | Loose stool |
| | 2 | Loose/some diarrhea |
| | 3 | Diarrhea |
| | 4 | Severe watery diarrhea |
| Posture | 2 | Normal body posture |
| | 1 | Moderately hunched posture |
| | 0 | Severely hunched posture |

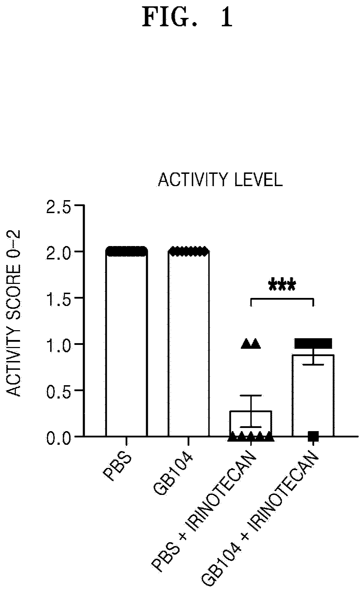

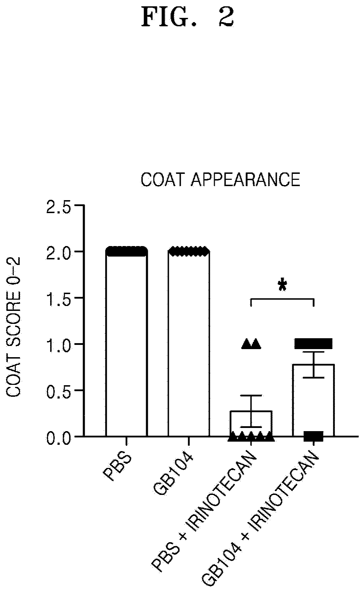

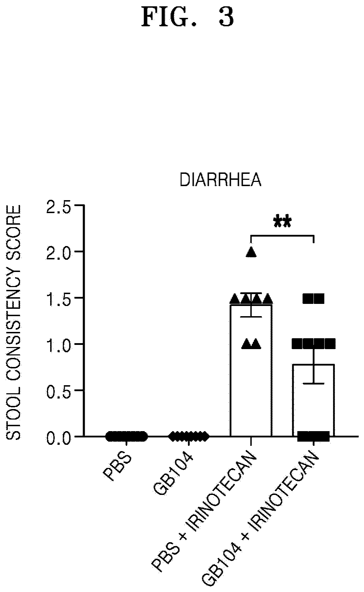

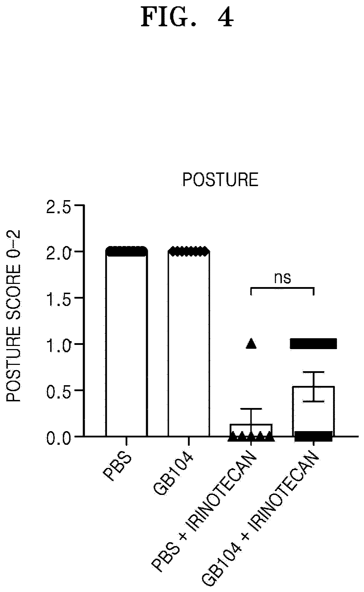

Mouse activity level was scored according to the amount each mouse moved in a cage (score 2: an animal moved around the case normally, score 1: an animal was moving slowly or less frequently and with an altered gait, and score 0: an animal was not moving and was taking no more than 5 steps), and the results thereof are illustrated in FIG. 1. The appearance of the coat was scored according to smoothness (score 2: a healthy, smooth uninterrupted coat, score 1: a slightly fluffy coat, and score 0: a severe fluffy coat with evident gaps of visible skin), and the results thereof are illustrated in FIG. 2. The severity of diarrhea was assessed according to the stool consistency score (0: normal, 1: loose stool, 2: loose/some diarrhea, 3: diarrhea, and 4: severe watery diarrhea), and the results thereof are illustrated in FIG. 3. Posture was scored as follows (score 2: a normal body posture, score 1: a moderately hunched posture, and score 0: a severely hunched posture), and the results thereof are illustrated in FIG. 4.

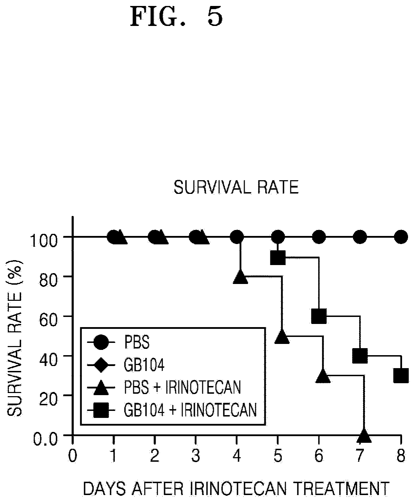

As illustrated in FIGS. 1 to 5, it was confirmed that, compared to G3 (PBS+Irinotecan), G4 (GB104+lrinotecan) alleviated various side effects such as decreased mouse activity and diarrhea induction caused by administration of a high dose of irinotecan, and contributed to improved survival rates.

### Experimental Example 2. Effect of L. plantarum GB104 strain on alleviating lethargy (decreased activity) in mouse colorectal cancer model with chemotherapy-induced diarrhea (CID)

The effect of the *L. plantarum* GB104 strain on alleviating lethargy (decreased activity) upon administration of a high dose of irinotecan was examined in a mouse colorectal cancer model with CID.

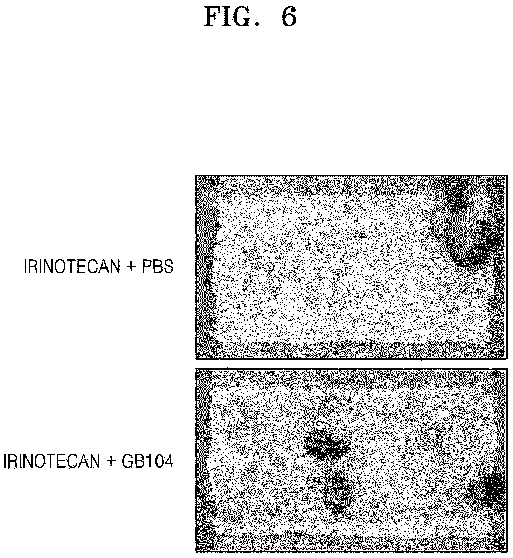

Five-week-old C57BL/6 mice were purchased and acclimatized for one week, and experiments were conducted at 6 weeks of age after hair removal from the right flank. A tumor model was established by subcutaneously injecting the C57BL/6-derived colorectal cancer cell line MC-38 into the right flank of each mouse at a concentration of 2 x 10⁵ cells/100 µL per mouse. Tumor volume was measured using a digital caliper and calculated by using tumor volume (mm³) (width² x length)/2. On day 5 after tumor cell injection, only mice with tumor volumes within the range of 10 to 30 mm³ were selected, and each group was randomly set and an experimental group was constructed in the same manner as in Experimental Example 1. Then, L. *plantarum* GB104 strain was orally administered daily to the animal model at a concentration of 1 x 10⁹ CFU per mouse from day 5 until immediately before the experiments were completed. The chemotherapeutic agent irinotecan (200 mg/kg) was administered intraperitoneally on days 9, 11, and 13 after tumor cell injection. To confirm the effect of the *L. plantarum* GB104 strain on decreased activity (lethargy), which is one of the side effects caused by administration of a high dose of irinotecan, the movement of mice in each group was analyzed by video tracking using the Ethovision XT program, and the results thereof are illustrated in FIG. 6.

As illustrated in FIG. 6, it was confirmed that the lethargy and decreased activity of mice upon administration of a high dose of irinotecan, confirmed in a negative control (PBS+Irinotecan), were alleviated in an experimental group (GB104+lrinotecan).

### Experimental Example 3. Effect of L. plantarum GB104 strain on alleviating colon tissue damage upon administration of high dose of irinotecan

The effect of the *L. plantarum* GB104 strain on alleviating colon tissue damage upon administration of a high dose of irinotecan was examined in a mouse colorectal cancer model with CID.

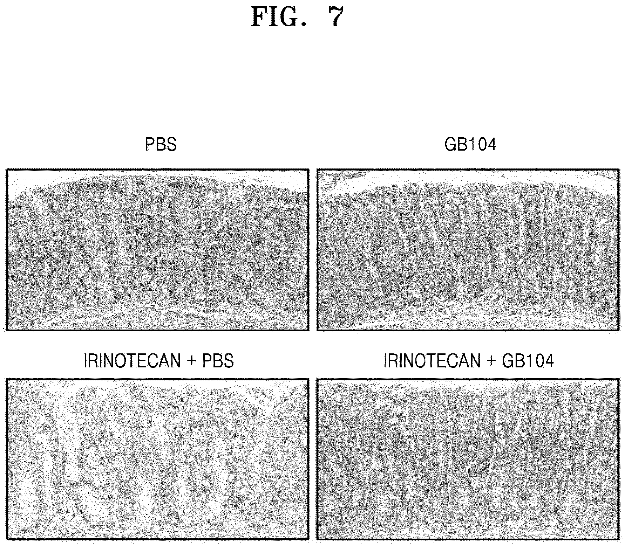

In the mouse colorectal cancer model with CID of Experimental Example 2, colon tissues were excised on day 19 after tumor cell injection and fixed in 10% formalin, and paraffin blocks were then prepared and sectioned to a thickness of 4 µm, and slides were prepared. Changes in colon tissues were examined through hematoxylin & eosin (H&E) staining, and the results thereof are illustrated in FIG. 7.

As illustrated in FIG. 7, it was confirmed that substantial damage to the intestinal crypts upon administration of a high dose of irinotecan was observed in the negative control (PBS+lrinotecan), whereas inflammatory responses in intestinal tissues and damage to colon tissues were alleviated in the experimental group (GB104+lrinotecan) administered with *L. plantarum* GB104 strain.

### Experimental Example 4. Effect of L. plantarum GB104 strain on strengthening tight junctions in intestinal tissues upon administration of high dose of irinotecan

The effect of the *L. plantarum* GB104 strain on strengthening tight junctions in colon tissues upon administration of a high dose of irinotecan was examined in a mouse colorectal cancer model with CID.

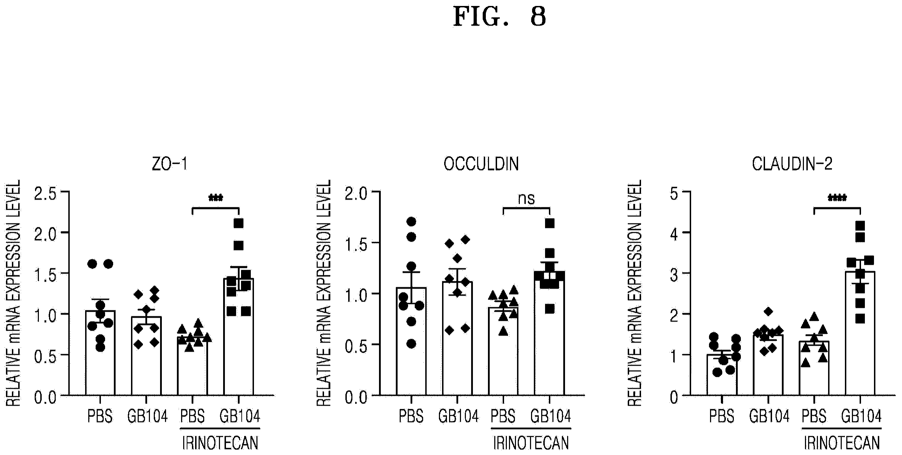

Five-week-old C57BL/6 mice were purchased and acclimatized for one week, and experiments were conducted at 6 weeks of age after hair removal from the right flank. A tumor model was established by subcutaneously injecting the C57BL/6-derived colorectal cancer cell line MC-38 into the right flank of each mouse at a concentration of 2 x 10⁵ cells/100 µL per mouse. Tumor volume was measured using a digital caliper and calculated by using tumor volume (mm³) (width² x length)/2. On day 5 after tumor cell injection, only mice with tumor volumes within the range of 10 to 30 mm³ were selected, and each group was randomly set and an experimental group was constructed in the same manner as in Experimental Example 1. Then, the L. *plantarum* GB104 strain was orally administered daily to the animal model at a concentration of 1 x 10⁹ CFU per mouse from day 5 until immediately before the experiments were completed. The chemotherapeutic agent irinotecan (50 mg/kg) was administered intraperitoneally on days 9, 11, 15, and 17 after tumor cell injection. On day 19 after tumor cell injection, colon tissues were excised and homogenized to isolate RNA, which was then synthesized into cDNA, and the mRNA expression levels of tight junction-associated genes (ZO-1, Occludin, Claudin-2) were determined using a QuantStudio 3 Real-Time PCR Instrument, and the results thereof are illustrated in FIG. 8.

As illustrated in FIG. 8, compared to the negative control (PBS+Irinotecan), the experimental group (GB104+lrinotecan) showed a significant increase in the mRNA expression levels of the tight junction-associated genes. Through these results, it was confirmed that the *L. plantarum* GB104 strain can alleviate side effects caused by administration of a high dose of an anticancer agent, including damage to intestinal epithelial cells, increased permeability, and weakened intestinal barrier integrity.

The foregoing description of the present disclosure is provided for illustrative purposes only, and it will be understood by those of ordinary skill in the art to which the present disclosure pertains that the present disclosure may be easily modified in other particular forms without changing the technical spirit or essential characteristics of the present disclosure. Therefore, the above-described embodiments should be construed as being provided for illustrative purposes only and not for purposes of limitation.

### [Accession number]

Name of depository institution: Korea Research Institute of Bioscience and Biotechnology
Accession number: KCTC14107BP
Accession date: January 14, 2020

## Claims

1. A composition for use as an adjuvant to anticancer treatment, comprising a *Lactobacillus plantarum* strain, a culture of the strain, a lysate of the strain, or a mixture thereof.

2. The composition of claim 1, wherein the composition is for reducing, ameliorating, preventing, or treating a side effect caused by an anticancer agent.

3. The composition of claim 1, wherein the strain comprises the 16S rRNA of SEQ ID NO: 1.

4. The composition of claim 1, wherein the strain is a strain deposited under accession number KCTC14107BP.

5. The composition of claim 1, wherein the strain comprises a mutation of a naturally occurring *Lactobacillus plantarum* strain.

6. The composition of claim 2, wherein the anticancer agent is any one selected from the group consisting of a chemotherapeutic agent, a targeted anticancer agent, and a cancer immunotherapeutic agent.

7. The composition of claim 6, wherein the anticancer agent is any one selected from the group consisting of mechlorethamine, cyclophosphamide, chlorambucil, ifosfamide, melphalan, thiotepa, altretamine, procarbazine, busulfan, streptozocin, carmustine, lomustine, dacarbazine, cisplatin, carboplatin, oxaliplatin, vinblastine, vincristine, vinorelbine, fluorouracil (5-FU), capecitabine, cytarabine, gemcitabine, fludarabine, methotrexate, pemetrexed, mercaptopurine (6-MP), topotecan, irinotecan, etoposide, paclitaxel, docetaxel, etoposide, dactinomycin, doxorubicin, daunorubicin, mitomycin, bleomycin, bleosein, bevacizumab, cetuximab, rituximab, trastuzumab, panitumumab, aflibercept, rituximab, pembrolizumab, nivolumab, atezolizumab, duvalumab, avelumab, ipilimumab, gefitinib, erlotinib, osimertinib, lazertinib, afatinib, crizotinib, alectinib, brigatinib, sorafenib, sunitinib, pazopanib, lenvatinib, axitinib, cabozantinib, imatinib, dasatinib, nilotinib, midostaurin, palbociclib, ribociclib, abemaciclib, olaparib, trifluridine/tipiracil, and regorafenib, or a combination thereof.

8. The composition of claim 2, wherein the side effect is selected from the group consisting of vomiting, oral mucositis, colitis, ulcerative colitis, diarrhea, enteritis-induced diarrhea, constipation, esophagitis, bleeding, alopecia, infection, fever, thrombocytopenia, anemia, abdominal pain, peripheral neurotoxicity, central neurotoxicity, muscle pain, bone pain, decreased activity, lethargy, decreased appetite, weight loss, fatigue, decreased food intake, nephrotoxicity, splenic toxicity, thymic toxicity, hepatotoxicity, cardiotoxicity, pulmonary toxicity, decreased motor function, immunotoxicity, and inflammation.

9. The composition of claim 1, wherein the cancer is any one selected from the group consisting of gastric cancer, colorectal cancer, pancreatic cancer, gallbladder cancer, biliary tract cancer, thyroid cancer, salivary gland cancer, esophageal cancer, head and neck cancer, small intestine cancer, anal cancer, colon cancer, rectal cancer, lung cancer, kidney cancer, breast cancer, and lymphoma.

10. The composition of claim 1, wherein the strain upregulates the expression of a tight junction in intestinal tissue.

11. The composition of claim 1, wherein the strain inhibits intestinal inflammatory responses or intestinal tissue damage induced by an anticancer agent.

12. The composition of claim 1 or 2, wherein the strain and the anticancer agent are co-administered simultaneously, sequentially, or in reverse order.

13. The composition of claim 1 or 2, wherein the strain and the anticancer agent are administered orally, intravenously, or subcutaneously.

14. The composition of claim 1 or 2, wherein the strain is administered orally, and the anticancer agent is administered subcutaneously.

15. A composition for enhancing sensitivity to an anticancer agent, the composition comprising a *Lactobacillus plantarum* strain, a culture of the strain, a lysate of the strain, or a mixture thereof.

16. An anticancer adjuvant comprising a *Lactobacillus plantarum* strain, a culture of the strain, a lysate of the strain, or a mixture thereof.

17. A method of preventing or reducing colon damage in a subject administered with an anticancer agent, the method comprising administering, to the subject administered with an anticancer agent, a *Lactobacillus plantarum* strain, a culture of the strain, a lysate of the strain, or a mixture thereof.

18. The method of claim 17, wherein the administration of the strain starts before the administration of the anticancer agent.

19. The method of claim 17, wherein the administration of the strain starts during the administration of the anticancer agent.

20. The method of claim 17, wherein the administration of the strain starts after the administration of the anticancer agent is completed.

21. Use of a composition comprising a *Lactobacillus plantarum* strain, a culture of the strain, a lysate of the strain, or a mixture thereof, as an adjuvant to anticancer treatment.

22. A method for use as an adjuvant to anticancer treatment, the method comprising administering an effective amount of a composition comprising a *Lactobacillus plantarum* strain, a culture of the strain, a lysate of the strain, or a mixture thereof to a subject in need thereof.

23. Use of a composition comprising a *Lactobacillus plantarum* strain, a culture of the strain, a lysate of the strain, or a mixture thereof for the preparation of a pharmaceutical formulation, as an adjuvant to anticancer treatment.
